# EUROPEAN PATENT APPLICATION

(11) **EP 4 283 628 A1**
(43) Date of publication of application: **29.11.2023**
(21) Application number: 21920987.1
(22) Date of filing: 21.01.2021
(51) Int. Cl.: G16H 20/70, G16H 50/30, A61B 5/16

(54) **INFORMATION PROCESSING APPARATUS, INFORMATION PROCESSING METHOD, AND PROGRAM**

(71) Applicant: Sony Group Corporation, Tokyo 108-0075 (JP)
(72) Inventor: TOKUTAKE, Kenji, Tokyo 140-0002 (JP); FUJITA, Shuji, Tokyo 108-0075 (JP); TAKAHASHI, Tomotaka, Tokyo 108-0075 (JP)
(74) Representative: 2SPL Patentanwälte PartG mbB
(86) International application number: PCT/JP2021/001976
(87) International publication number: WO 2022/157874

(57) **Abstract**

An information processing apparatus includes an estimation unit that estimates stress applied to a user based on schedule information of the user, and a determination unit that makes a determination of a feedback related to a schedule of the user based on an estimation result of the stress.

## Description

### Field

The present disclosure relates to an information processing apparatus, an information processing method, and a program.

### Background

Stress applied to a person is becoming a concern. In particular, in recent years, since a home-based working has been expanded, it is difficult to see from the outside how much stress is applied to a person. The stress is becoming a serious concern because, for example, it is too late when the stress becomes apparent.

### Citation List

### Patent Literature

Patent Literature 1: JP 2018-011721 A
Patent Literature 2: JP 2019-067151 A
Patent Literature 3: JP 2016-120224 A
Patent Literature 4: JP 2020-029108 A

### Summary

### Technical Problem

It is possible to estimate to some extent how much stress is applied to a person using a computer (and a sensor). However, although the stress can be estimated, the stress does not reduce just by estimation.

Therefore, the present disclosure proposes an information processing apparatus, an information processing method, and a program that can contribute to stress reduction.

### Solution to Problem

In order to solve the above problem, an information processing apparatus according to one aspect of the present disclosure includes: an estimation unit configured to estimate stress applied to a user based on schedule information of the user; and a determination unit configured to make a determination of a feedback related to a schedule of the user based on an estimation result of the stress.

### Brief Description of Drawings

FIG. 1 is a diagram illustrating a change in pre-COVID-19 and post-COVID-19 working conditions.
FIG. 2 is a diagram illustrating an outline of a present embodiment.
FIG. 3 is a diagram illustrating an effect when a brief meditation is implemented.
FIG. 4 is a diagram illustrating an outline of the present embodiment.
FIG. 5 is a diagram illustrating an example of communication using AI.
FIG. 6 is a diagram illustrating another example of communication using AI.
FIG. 7 is a diagram illustrating a configuration example of a management system according to the embodiment of the present disclosure.
FIG. 8 is a diagram illustrating a configuration example of a management server according to the embodiment of the present disclosure.
FIG. 9 is a diagram illustrating a configuration example of a terminal device according to the embodiment of the present disclosure.
FIG. 10 is a flowchart illustrating a first learning process according to the present embodiment.
FIG. 11 is a diagram illustrating an actual measurement result of stress of a user.
FIG. 12 is a flowchart illustrating a feedback process according to the present embodiment.
FIG. 13 is a diagram illustrating event setting in a home-based working style.
FIG. 14 is a diagram illustrating event setting in an office-based working style.
FIG. 15 is a flowchart illustrating a second learning process according to the present embodiment.
FIG. 16 is a diagram illustrating data acquired by the management server for training a learning model.
FIG. 17 is a diagram illustrating an example of a process when a care target is a group/a plurality of persons.
FIG. 18 is a diagram illustrating another example of a process when the care target is a group/a plurality of persons.

### Description of Embodiments

Hereinafter, embodiments of the present disclosure will be described with reference to the drawings. In each of the following embodiments, same parts are given the same reference signs to omit redundant description.

In addition, in the present specification and the drawings, a plurality of components having substantially the same functional configuration may be distinguished by attaching different numerals after the same reference sign. For example, a plurality of configurations having substantially the same functional configuration is distinguished as terminal devices 20₁, 20₂, and 20₃ as necessary. However, when it is not particularly necessary to distinguish each of the plurality of components having substantially the same functional configuration, only the same reference sign is given. For example, when it is not necessary to particularly distinguish the terminal devices 20₁, 20₂, and 20₃, they are simply referred to as a terminal device 20.

The present disclosure will be described according to the following item order.
1. Outline of present embodiment
2. Configuration of management system
   2-1. Configuration of management server
   2-2. Configuration of terminal device
   2-3. Learning model
3. Operation of management system
   3-1. First learning process
   3-2. Feedback process
   3-3. Second learning process
   3-4. Process when a care target is a group/a plurality of persons
4. Modifications
5. Conclusion

### <<1. Outline of present embodiment>>

In recent years, due to the spread of the novel coronavirus infection, an office-based working style before the spread of the infection (hereinafter referred to as pre-COVID-19), has changed to a telework style after the spread of the infection (hereinafter referred to as post-COVID-19). An expansion of the home-based working style makes it difficult to see how much stress is applied to a person. In addition, how a person is stressed also changes between the office-based working style and the home-based working style.

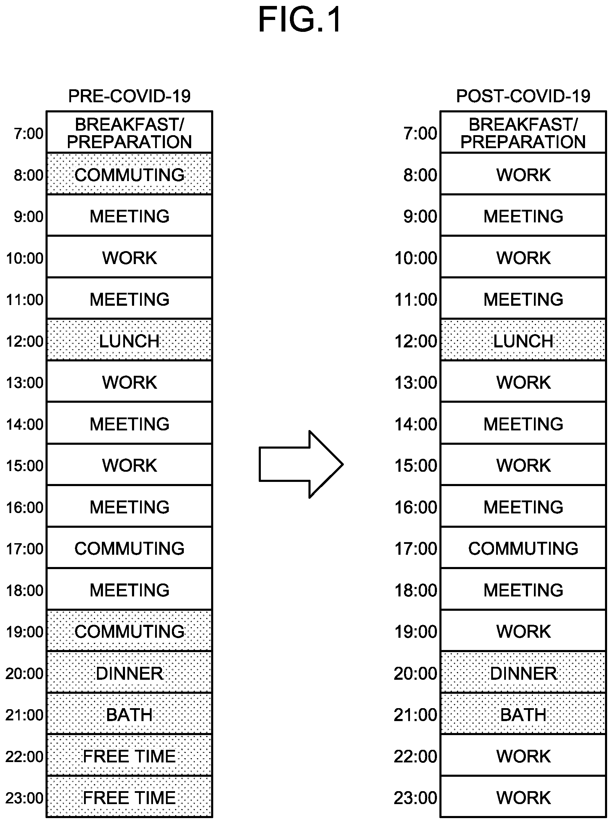

FIG. 1 is a diagram illustrating a change in pre-COVID-19 and post-COVID-19 working conditions. In FIG. 1, a schedule illustrated on the left side is an example of a pre-COVID-19 schedule, and a schedule illustrated on the right side is an example of a post-COVID-19 schedule.

In the pre-COVID-19 office-based working style, a commuting time naturally occurs. In the example in FIG. 1, the commuting time occurs between 8:00 and 9:00 and between 19:00 and 20:00. In addition, in the office-based working style, it is considered that work is rarely performed after returning home and finishing dinner and a bath. Also in the example in FIG. 1, there is free time between 22:00 and 23:00 and between 23:00 and 24:00 after the dinner and the bath.

On the other hand, the commuting time naturally does not occur in the post-COVID-19 home-based working style. In the example in FIG. 1, the commuting time between 8:00 and 9:00 and between 19:00 and 20:00 has changed to the work time. In addition, since it is difficult to distinguish between ON and OFF of work in the home-based working style, it is considered that there are many cases where work is performed after finishing dinner and a bath. In the example in FIG. 1, the free time between 22:00 and 23:00 and between 23:00 and 24:00 has changed to the work time. In other words, in the example in FIG. 1, not only the commuting time but also time after finishing dinner and a bath are spent for working. Therefore, in the example in FIG. 1, it is assumed that an excessive stress is applied to a user.

In the home-based working style, even when the excessive stress is applied to the user due to increased work time, it is difficult to notice it from outside. When the state of excessive stress continues, a work efficiency naturally decreases, and in some cases, it becomes too late to improve the situation.

It is possible to estimate to some extent how much stress is applied to a person using an information processing apparatus (computer) and a sensor. However, although the stress can be estimated, the stress does not reduce just by estimation. In addition, a cause of stress cannot be identified.

In order to reduce stress, it is considered that a short meditation (hereinafter also referred to as a brief meditation) during work or before and after work is effective. For example, it is considered that concentration is greatly restored by five minutes of meditation. Furthermore, it is considered that communication during work or before and after work is also effective for reducing stress. In particular, when there is no schedule and communication is insufficient, when the worker works at home alone, or when the worker has hidden depression, it is assumed that implementation of communication has an extremely large effect on stress reduction.

Therefore, in the present embodiment, the information processing apparatus estimates stress applied to the user based on schedule information of the user. Then, the information processing apparatus determines a feedback related to the schedule of the user based on a stress estimation result. The feedback may be, for example, a schedule setting for changing, adding, or deleting the schedule of the user. For example, the information processing apparatus sets an event for reducing stress (e.g., brief meditation or communication) in the schedule of the user. In addition, from a change of the stress estimation result, an event for stress reduction may be set before and after the schedule (cause) where the stress greatly changes.

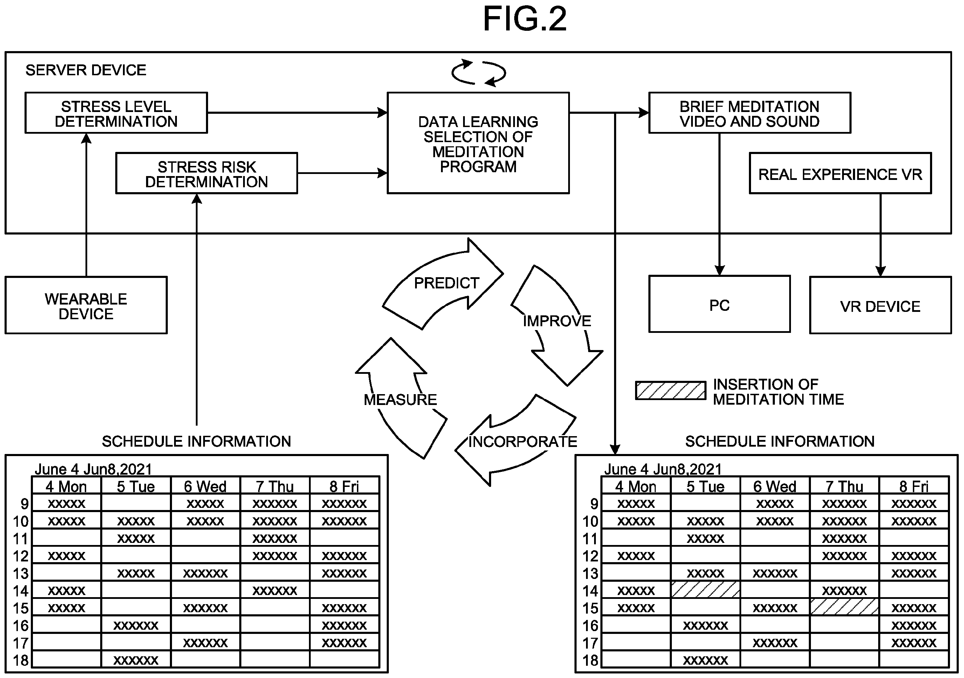

FIG. 2 is a diagram illustrating an outline of the present embodiment. In an example in FIG. 2, a server device, which is an example of the information processing apparatus, estimates the stress applied to the user and inserts meditation time in the schedule of the user according to a degree of stress.

For example, the server device determines a stress risk of the user based on the schedule information of the user. Note that the server device may determine a stress level of the user based on a sensor value (pulse rate or the like) of a wearable device. Then, the server device learns a relationship between the schedule and the stress based on a determination result. Then, when it is estimated that high stress is applied to the user, the server device selects a meditation program suitable for a stress state. The meditation program may be a video or a sound for the brief meditation, or may be a real experience VR using a VR device. Then, the server device inserts the meditation time into the schedule of the user.

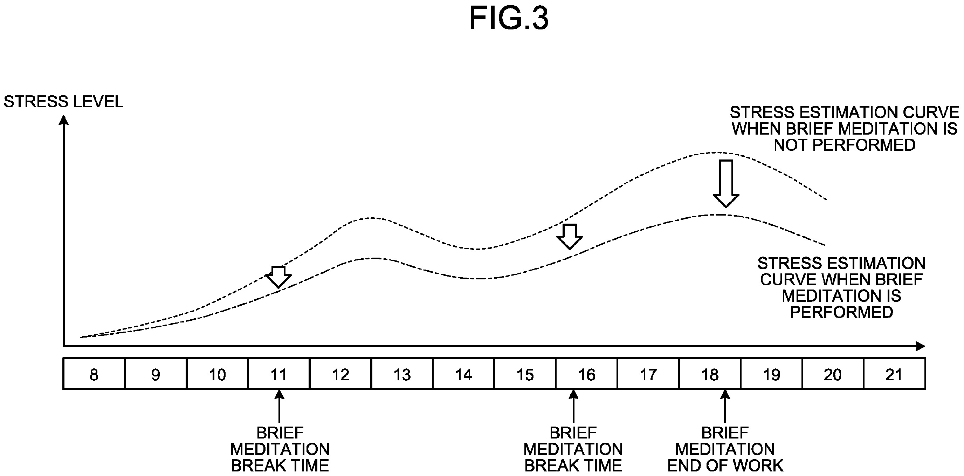

FIG. 3 is a diagram illustrating an effect when the brief meditation is implemented. More specifically, FIG. 3 is a diagram illustrating a change in a stress estimation curve between a case where the brief meditation is not implemented and a case where the brief meditation is implemented. The stress estimation curve is a graph illustrating a stress estimation result of the user. The stress estimation result is generated using, for example, a learning model that has learned the relationship between the schedule and the stress. In the example in FIG. 3, the brief meditation is inserted at a break between 11:00 and 12:00 and between 16:00 and 17:00, and the brief meditation is inserted at the end of work between 18:00 and 19:00. As illustrated in FIG. 3, when the brief meditation is implemented, the stress is greatly reduced, and the stress estimation curve is greatly shifted downward (stress reducing direction).

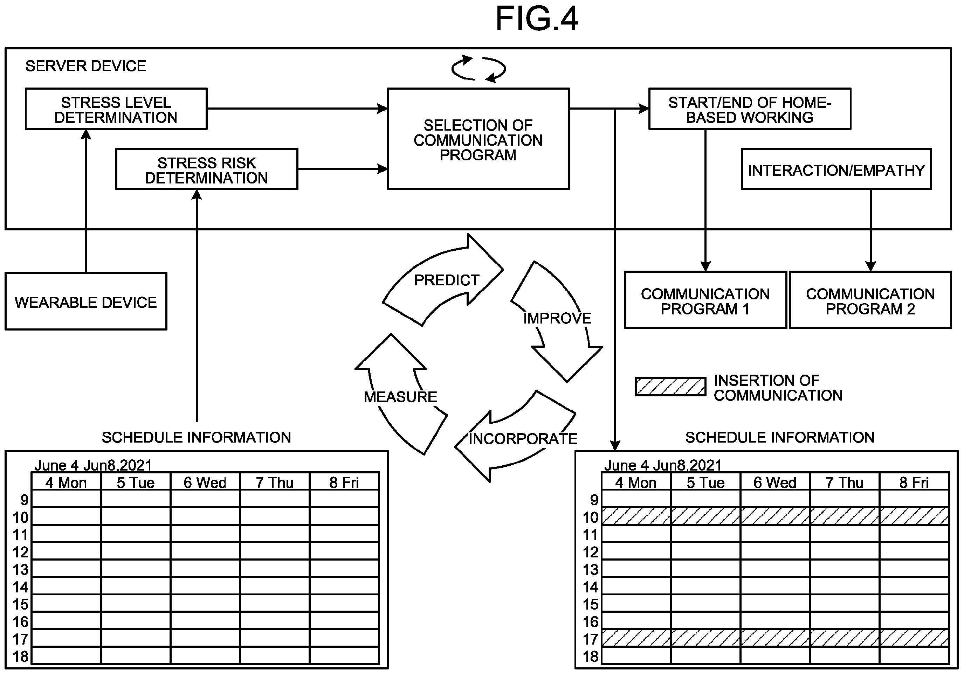

FIG. 4 is a diagram illustrating an outline of the present embodiment. In the example in FIG. 4, the server device estimates the stress applied to the user and inserts communication time into the schedule of the user.

For example, the server device determines a stress risk of the user based on the schedule information of the user. Note that the server device may determine the stress level of the user based on a sensor value (pulse rate or the like) of the wearable device. Then, the server device selects a communication program suitable for the state of the user based on the determination result. For example, the server device selects a communication program 1 at the start/end of the home-based working. On the other hand, the server device selects a communication program 2 when it is determined that the user is in a situation requiring interaction or empathy. The communication program may use artificial intelligence (AI). For example, the communication may be communication by a virtual character such as an avatar. Then, the server device inserts the communication time into the schedule of the user.

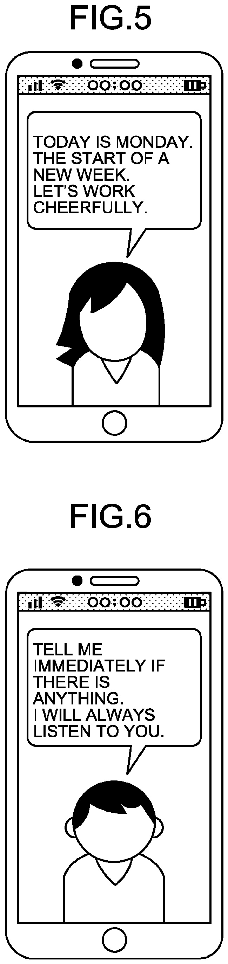

FIG. 5 is a diagram illustrating an example of communication using AI. The server device distinguishes business start time based on the schedule information of the user, and transmits the communication program using the virtual character to the terminal device of the user at predetermined time before the business start time. Then, the terminal device executes the communication program when the business start time comes. In the example in FIG. 5, the virtual character says to the user "Today is Monday. The start of a new week. Let's work cheerfully". The communication program may be configured to respond to a question from the user.

FIG. 6 is a diagram illustrating another example of communication using AI. The server device determines a time when stress increases based on the schedule information of the user, and transmits the communication program using the virtual character to the terminal device of the user at a predetermined time before the determined time. Then, when the determined time comes, the terminal device executes the communication program. In the example in FIG. 6, the virtual character says to the user, "Tell me immediately if there is anything. I will always listen to you". The communication program may be configured to respond to a question from the user. Furthermore, when a stress value greatly changes, understanding of user's stress characteristic may be deepened by performing training for stress estimation together with stress reduction by identifying a cause via the communication program.

According to the information processing apparatus of the present embodiment, feedback for reducing stress can be performed in view of the schedule information. For example, the information processing apparatus can insert an event such as the brief meditation or communication into the schedule of the user in order to reduce the stress of the user. The stress of the user can be reduced by the user implementing the event. Furthermore, by executing the communication program at the start/end of work, it is possible to make the user aware of the start/end time of work through communication, and it is possible to suppress long work of the user and encourage a style of clearly dividing work and private time.

The outline of the present embodiment has been described above, and a management system 1 according to the present embodiment will be described in detail below.

### <<2. Configuration of management system>>

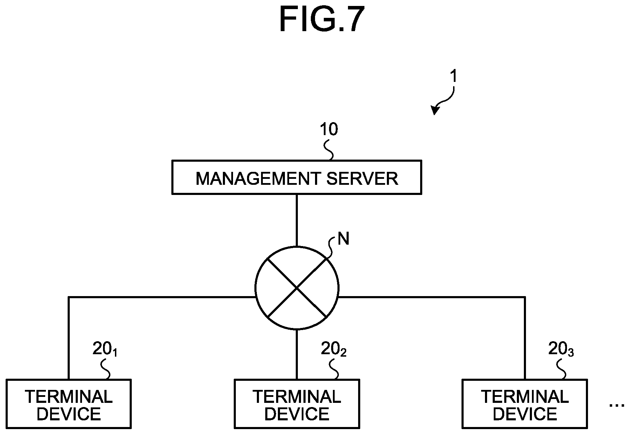

FIG. 7 is a diagram illustrating a configuration example of the management system 1 according to the embodiment of the present disclosure. The management system 1 is an information processing system that performs various processes related to the user, such as user schedule management.

The management system 1 includes a management server 10 and a terminal device 20. Note that devices in the drawings may be considered as devices in a logical sense. In other words, a part of the devices in the drawing may be realized by a virtual machine (VM), a container, a docker, or the like, and they may be implemented on physically the same hardware.

The management server 10 and the terminal device 20 each have a communication function and are connected via a network N. In the following description, the management server 10 and the terminal device 20 may be referred to as communication devices. Although only one network N is illustrated in the example in FIG. 7, a plurality of networks N may exist.

The network N is, for example, a communication network such as a local area network (LAN), a wide area network (WAN), a cellular network, a fixed telephone network, a regional Internet protocol (IP) network, or the Internet. The networks N may include a wired network or a wireless network. In addition, the network N may include a core network. The core network is, for example, an evolved packet core (EPC) or a 5G core network (5GC). In addition, the network N may include a data network other than the core network. The data network may also be a service network of a telecommunications carrier such as an IP multimedia subsystem (IMS) network. Furthermore, the data network may be a private network such as an intra-company network.

The communication device such as the terminal device 20 may be configured to connect to the network N using a radio access technology (RAT) such as long term evolution (LTE), new radio (NR), Wi-Fi, and Bluetooth (registered trademark). In this case, the communication device may be configured to be able to use different radio access technologies. For example, the communication device may be configured to be able to use the NR and the Wi-Fi. Furthermore, the communication device may be configured to be able to use different cellular communication technologies (e.g., LTE and NR). The LTE and NR are types of cellular communication technology, and enable mobile communication of the communication device such as the terminal device 20 by arranging a plurality of cellular areas covered by a base station.

Hereinafter, a configuration of each device configuring the management system 1 will be specifically described. Note that the configuration of each device described below is merely an example. The configuration of each device may be different from the following configuration.

### <2-1. Configuration of management server>

First, a configuration of the management server 10 will be described.

The management server 10 is an information processing apparatus (computer) that performs processing related to stress of the user, such as estimation of stress applied to the user and feedback to the user. The management server 10 may be a PC server, a midrange server, or a mainframe server.

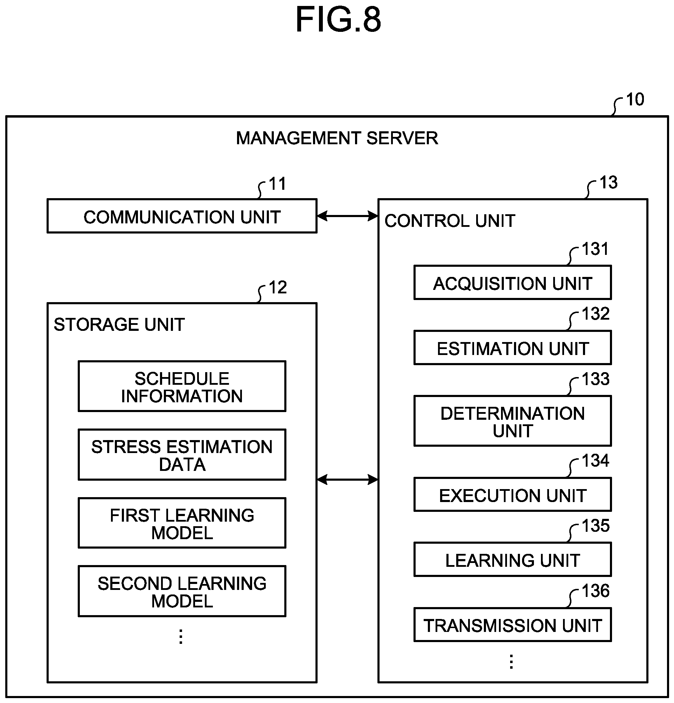

FIG. 8 is a diagram illustrating a configuration example of the management server 10 according to the embodiment of the present disclosure. The management server 10 includes a communication unit 11, a storage unit 12, and a control unit 13. Note that the configuration illustrated in FIG. 8 is a functional configuration, and a hardware configuration may be different from the functional configuration. Furthermore, functions of the management server 10 may be implemented in a distributed manner in a plurality of physically separated configurations. For example, the management server 10 may include a plurality of server devices.

The communication unit 11 is a communication interface for communicating with other devices. For example, the communication unit 11 is a local area network (LAN) interface such as a network interface card (NIC). The communication unit 11 may be a wired interface or a wireless interface. The communication unit 11 communicates with the terminal device 20 according to the control of the control unit 13.

The storage unit 12 is a data readable/writable storage device such as a dynamic random access memory (DRAM), a static random access memory (SRAM), a flash memory, or a hard disk. The storage unit 12 functions as a storage means of the management server 10. The storage unit 12 stores, for example, various types of information regarding the user. For example, the storage unit 12 stores the schedule information of the user and stress estimation data of the user. In addition, the storage unit 12 stores learning models such as a first learning model and a second learning model. The learning models will be described in detail later.

The control unit 13 is a controller that controls each unit of the management server 10. The control unit 13 is realized by, for example, a processor such as a central processing unit (CPU) or a micro processing unit (MPU). For example, the control unit 13 is implemented by a processor executing various programs stored in a storage device inside the management server 10 using a random access memory (RAM) or the like as a work area. Note that the control unit 13 may be realized by an integrated circuit such as an application specific integrated circuit (ASIC) or a field programmable gate array (FPGA). Any of the CPU, the MPU, the ASIC, and the FPGA can be regarded as the controller. Furthermore, the control unit 13 may be realized by the GPU in addition to or instead of the CPU.

The control unit 13 includes an acquisition unit 131, an estimation unit 132, a determination unit 133, an execution unit 134, a learning unit 135, and a transmission unit 136. Each block (the acquisition unit 131 to the transmission unit 136) configuring the control unit 13 is a functional block indicating a function of the control unit 13. These functional blocks may be software blocks or hardware blocks. For example, each of the functional blocks described above may be one software module realized by software (microprogram), or may be one circuit block on a semiconductor chip (die). It is apparent that each functional block may be one processor or one integrated circuit. The control unit 13 may be configured by a functional unit different from the above-described functional blocks. A configuration method of the functional blocks is arbitrary.

Note that the control unit 13 may be configured by a functional unit different from the above-described functional block. In addition, some or all of the operations of the blocks (the acquisition unit 131 to the transmission unit 136) configuring the control unit 13 may be performed by another device. For example, some or all of the operations of the blocks constituting the control unit 13 may be performed by a control unit 23 of the terminal device 20. The operation of each block configuring the control unit 13 will be described later.

### <2-2. Configuration of terminal device>

Next, a configuration of the terminal device 20 will be described.

The terminal device 20 is a communication device that communicates with the management server 10. For example, the terminal device 20 is a terminal possessed by the user whose schedule is managed using the management server 10. The terminal device 20 exchanges various types of information regarding the user with the management server 10. For example, the terminal device 20 exchanges the schedule information of the user and biometric information of the user with the management server 10.

For example, the terminal device 20 may be a mobile phone, a smart device (smartphone or tablet), a personal digital assistant (PDA), or a personal computer. Still more, the terminal device 20 may be an imaging device (e.g., camcorder) having a communication function, or may be a moving object (e.g., a motorcycle and a moving relay vehicle) equipped with a communication apparatus such as a field pickup unit (FPU). Furthermore, the terminal device 20 may be a machine to machine (M2M) device or an Internet of things (IoT) device.

Still more, the terminal device 20 may be an xR device such as an augmented reality (AR) device, a virtual reality (VR) device, and a mixed reality (MR) device. Here, the xR device may be a glasses-type device (e.g., AR/MR/VR glasses) or a head-mounted or goggle-type device (e.g., AR/MR/VR headsets and AR/MR/VR goggles). These xR devices may display a video for only one eye or may display a video for both eyes.

Still more, the terminal device 20 may be a wearable device such as a smart watch. Here, the wearable device may be configured to be able to acquire information regarding the user wearing the device. For example, the wearable device may be configured to be able to acquire the biological information of the user such as a body temperature (e.g., skin temperature), electro dermal activity (EDA), and pulse.

Furthermore, the terminal device 20 may be a device that acquires information regarding an environment in which the user is placed (hereinafter referred to as environment information). For example, the terminal device 20 may be a measurement device that measures the environment information. The environment information is, for example, an indoor temperature, a humidity, and a CO₂ concentration. Note that the measurement device may be a part of the terminal device 20.

Furthermore, the terminal device 20 may be an audio apparatus such as an earphone or a headphone. Note that the audio apparatus may be a part of the terminal device 20. For example, when the audio apparatus is a part of the xR device, the audio device may be configured such that the user can simultaneously listen to virtual sound and sound in the real space. As a result, since the user can continue working while wearing the audio apparatus, the user does not miss the stress reduction event using the audio apparatus. Furthermore, the audio apparatus may have a function of canceling sound (environmental sound) generated in a real space. This allows the user to focus on the stress reduction event using the audio apparatus. In the following description, a technique for canceling the environmental sound may be referred to as a noise canceling technique.

Furthermore, the terminal device 20 may be an aroma device having a function of reproducing aroma. The aroma device can be rephrased as an aroma reproducing device, an aroma generating device, or an aroma presentation device. Note that the aroma device may be a part of the terminal device 20. The presentation of the aroma can enhance the user's sense of immersion in the stress reduction event. In the following description, a technique for reproducing the aroma may be referred to as an aroma reproduction technique.

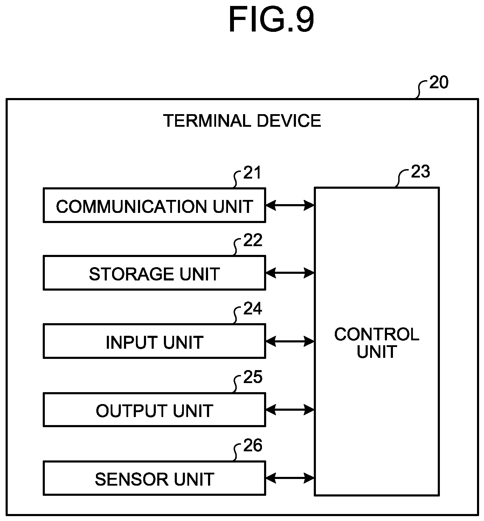

FIG. 9 is a diagram illustrating a configuration example of the terminal device 20 according to the embodiment of the present disclosure. The terminal device 20 includes a communication unit 21, a storage unit 22, the control unit 23, an input unit 24, an output unit 25, and a sensor unit 26. Note that the configuration illustrated in FIG. 9 is a functional configuration, and a hardware configuration may be different from the functional configuration. Furthermore, functions of the terminal device 20 may be implemented in a distributed manner in a plurality of physically separated configurations.

The communication unit 21 is a communication interface for communicating with other devices. For example, the communication unit 21 is a LAN interface such as the NIC. Note that the communication unit 21 may be a wired interface or a wireless interface. The communication unit 21 communicates with the management server 10 and the like according to the control of the control unit 23.

The storage unit 22 is a storage device capable of reading and writing data, such as a DRAM, an SRAM, a flash memory, or a hard disk.

The control unit 23 is a controller that controls each unit of the terminal device 20. The control unit 23 is realized by, for example, a processor such as the CPU or the MPU. For example, the control unit 23 is realized by a processor executing various programs stored in a storage device inside the terminal device 20 using the RAM or the like as a work area. Note that the control unit 23 may be realized by an integrated circuit such as the ASIC or the FPGA. Any of the CPU, the MPU, the ASIC, and the FPGA can be regarded as the controller. Furthermore, the control unit 23 may be realized by the GPU in addition to or instead of the CPU.

The input unit 24 is an input device that receives various inputs from outside. For example, the input unit 24 is an operation device for the user to perform various operations, such as a keyboard, a mouse, and an operation key. Note that, when a touch panel is adopted as the terminal device 20, the touch panel is also included in the input unit 24. In this case, the user performs various operations by touching a screen with a finger or a stylus.

The output unit 25 is a device that performs various outputs such as sound, light, vibration, and an image to outside. The output unit 25 includes a display device that displays various types of information. The display device is, for example, a liquid crystal display or an organic electro luminescence (EL) display. Note that, when the touch panel is adopted as the terminal device 20, the display device may be a device integrated with the input unit 24. The output unit 25 performs various outputs to the user according to the control of the control unit 23.

The sensor unit 26 is a sensor that acquires the biological information or the environment information. For example, the sensor unit 26 may be a biological sensor such as a thermometer, an electrodermal activity meter, or a pulsometer, or may be an environmental sensor such as a thermometer, a hygrometer, or a CO₂ concentration meter.

### <2-3. Learning model>

Next, the learning model will be described.

As described above, the storage unit 12 of the management server 10 stores the learning models such as the first learning model and the second learning model. The learning model is configured to be able to capture features of a schedule or a plan registered in the schedule, such as details of the plan registered in the schedule and a degree of schedule tightness. Then, the management server 10 estimates the stress of the user in the schedule, for example, using the learning model. Note that, in the following description, the first learning model and the second learning model are simply referred to as the learning model when it is not particularly necessary to distinguish the first learning model and the second learning model.

The learning model is, for example, a machine learning model such as a neural network model. The neural network model includes layers called an input layer including a plurality of nodes, an intermediate layer (or hidden layer.), and an output layer, and the nodes are connected via edges. Each layer has a function called an activation function, and each edge is weighted. The learning model has one or more intermediate layers (or hidden layers). When the learning model is the neural network model, training of the learning model means, for example, setting the number of intermediate layers (or hidden layers), the number of nodes in each layer, the weight of each edge, and the like.

Here, the neural network model may be a model generated by deep learning. In this case, the neural network model may be a model in a form called a deep neural network (DNN). The neural network model may be a model in a form called convolution neural network (CNN), recurrent neural network (RNN), or long short-term memory (LSTM). Naturally, the neural network model is not limited to these models.

Still more, the learning model is not limited to the neural network model. For example, the learning model may be a model using reinforcement learning. In the reinforcement learning, an action (setting) that maximizes a value is learned through trial and error.

Note that the learning model may include a plurality of models. For example, the learning model may include a plurality of neural network models. More specifically, the learning model may include, for example, a plurality of neural network models selected from the CNN, the RNN, and the LSTM. In a case where the learning model includes a plurality of neural network models, the plurality of neural network models may be in a dependency relationship or a parallel relationship.

As described above, the storage unit 12 of the management server 10 stores the learning models such as the first learning model and the second learning model. The first learning model and the second learning model are used in a feedback process to be described later. The feedback process is a process of determining feedback (e.g., schedule setting for stress reduction) to the user based on the stress estimation result of the user. Hereinafter, the first learning model and the second learning model will be described in detail.

The first learning model is a trained model that has learned the relationship between the schedule of the user and the stress applied to the user. For example, the first learning model is a model that receives at least information regarding the schedule of the user (hereinafter simply referred to as the schedule information) as an input and at least information regarding stress applied to the user (hereinafter simply referred to as stress information) is output.

Furthermore, the second learning model is a trained model that has learned a relationship between feedback to the user regarding the schedule of the user and an effect of reducing stress of the user when the feedback is provided. For example, the second learning model is a model in which information regarding feedback to the user (hereinafter simply referred to as feedback information) and the stress estimation result of the user in the schedule before the feedback is provided (hereinafter simply referred to as pre-feedback stress estimation result) are input, and the stress estimation result of the user in the schedule after the feedback is provided (hereinafter simply referred to as a post-feedback stress estimation result.) is output.

Note that, in the example in FIG. 8, character information such as the "first learning model" and the "second learning model" is described as the information stored in the storage unit 12. However, a character string, a numerical value, or the like indicating a structure or a connection coefficient of the model is actually stored in the storage unit 12.

The first learning model may be a model trained to output the stress information when the schedule information is input using, as learning data, a data set of the schedule information and actual measurement data of the stress of the user (hereinafter referred to as first actual measurement data) in the schedule indicated by the schedule information. In this case, the first learning model may be a model that includes the input layer to which the schedule information is input, the output layer that outputs the stress information, a first element that belongs to any layer from the input layer to the output layer other than the output layer, and a second element whose value is calculated based on the first element and the weight of the first element. The first learning model causes a computer to function to output the stress information from the output layer according to the schedule information input to the input layer by performing an operation based on the first element and the weight (e.g., connection coefficient) of the first element with each element belonging to each layer other than the output layer as the first element with respect to the information input to the input layer.

Furthermore, the second learning model may be a model trained to output the post-feedback stress estimation result when the feedback information and the pre-feedback stress estimation result are input using, as learning data, a data set of the feedback information, the pre-feedback stress estimation result, and actual measurement data of the stress of the user in the schedule after the feedback is provided (hereinafter referred to as second actual measurement data). In this case, the second learning model may be a model that includes the input layer to which the feedback information and the pre-feedback stress estimation result are input, the output layer that outputs the post-feedback stress estimation result, the first element that belongs to any layer from the input layer to the output layer other than the output layer, and the second element whose value is calculated based on the first element and the weight of the first element. The second learning model causes the computer to function to output the post-feedback stress estimation result from the output layer according to the information input to the input layer by performing an operation based on the first element and the weight (e.g., connection coefficient) of the first element with each element belonging to each layer other than the output layer as the first element with respect to the information input to the input layer.

Here, it is assumed that the learning model is realized by a neural network having one or a plurality of intermediate layers such as a DNN. In this case, the first element included in the learning model corresponds to any node included in the input layer or the intermediate layer. In addition, the second element corresponds to a node of a next stage that is a node to which a value is transmitted from the node corresponding to the first element. In addition, the weight of the first element corresponds to the connection coefficient that is a weight considered for a value transmitted from the node corresponding to the first element to the node corresponding to the second element.

Here, it is assumed that the learning model according to the present embodiment is realized by a regression model indicated by "y = a1 * x1 + a2 * x2 + · · · + ai * xi". In this case, the first element included in the learning model corresponds to input data (xi) such as x1 and x2. Further, the weight of the first element corresponds to a coefficient ai corresponding to xi. Here, the regression model can be regarded as a simple perceptron having the input layer and the output layer. When each model is regarded as the simple perceptron, the first element can be regarded as any node included in the input layer, and the second element can be regarded as a node included in the output layer.

The management server 10 calculates information to be output using a model having an arbitrary structure such as the neural network or the regression model. Specifically, in the first learning model, the coefficient is set so as to output the stress information when the schedule information is input. For example, the management server 10 sets the coefficient based on a similarity between the first actual measurement data and a value obtained by inputting the schedule information to the learning model. The management server 10 generates the stress information from the schedule information using such a learning model.

In addition, in the second learning model, the coefficient is set so as to output the post-feedback stress estimation result when the feedback information and the pre-feedback stress estimation result are input. For example, the management server 10 sets the coefficient so that the second actual measurement data approaches a value obtained by inputting the feedback information and the pre-feedback stress estimation result to the learning model. Using such a learning model, the management server 10 generates the post-feedback stress estimation result from the feedback information and the pre-feedback stress estimation result.

Note that, in the above example, as an example of the first learning model, the model that outputs the stress information when the schedule information is input has been described. Alternatively, the above example indicates, as the example of the second learning model, the model that outputs the post-feedback stress estimation result when the feedback information and the pre-feedback stress estimation result are input. However, the learning model according to the embodiment may be a model generated based on a result obtained by repeating input and output of data to and from the first learning model or the second learning model.

Furthermore, when the management server 10 performs learning using a generative adversarial network (GAN) or generates output information, the learning model may be a model constituting a part of the GAN.

Note that the learning device that trains the learning model (e.g., the first learning model and the second learning model) may be the management server 10 or another information processing apparatus. When the management server 10 trains the learning model, the learning unit 135 may train the learning model.

For example, it is assumed that the learning unit 135 of the management server 10 trains the learning model. In this case, the learning unit 135 trains the learning model and stores the trained learning model in the storage unit 12. More specifically, the learning unit 135 sets the connection coefficient of the learning model such that the learning model outputs the stress information when the schedule information is input to the learning model. Alternatively, the learning unit 135 sets the connection coefficient of the learning model such that the learning model outputs the post-feedback stress estimation result when the feedback information and the pre-feedback stress estimation result are input.

For example, the learning unit 135 inputs the schedule information to the node of the input layer included in the learning model, and causes the stress information to be output by propagating data to the output layer of the learning model through each intermediate layer. Then, the learning unit 135 corrects the connection coefficient of the learning model based on a difference between the stress information actually output by the learning model and the first actual measurement data. For example, the learning unit 135 may correct the connection coefficient using a method such as back propagation. At this time, the learning unit 135 may correct the connection coefficient based on a cosine similarity between a vector indicating the first actual measurement data and a vector indicating the value actually output by the learning model.

Furthermore, the learning unit 135 inputs the feedback information and the pre-feedback stress estimation result to the node of the input layer included in the learning model, and causes the post-feedback stress estimation result to be output by propagating data to the output layer of the learning model through each intermediate layer. Then, the learning unit 135 corrects the connection coefficient of the learning model based on a difference between a value actually output by the learning model and the second actual measurement data. For example, the learning unit 135 may correct the connection coefficient using a method such as back propagation. At this time, the learning unit 135 may correct the connection coefficient based on the cosine similarity between a vector indicating the second actual measurement data and a vector indicating the value actually output by the learning model.

Note that the learning unit 135 may train the learning model using any learning algorithm. For example, the learning unit 135 may train the learning model using the learning algorithm such as the neural network, a support vector machine, clustering, or the reinforcement learning.

### «3. Operation of management system»

The configuration of the management system 1 has been described above. Next, the operation of the management system 1 will be described. The operation of the management system 1 will be described in the following order (1) to (4).

### (1) First learning process

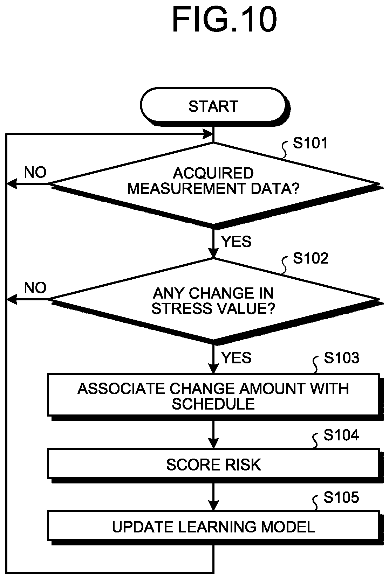

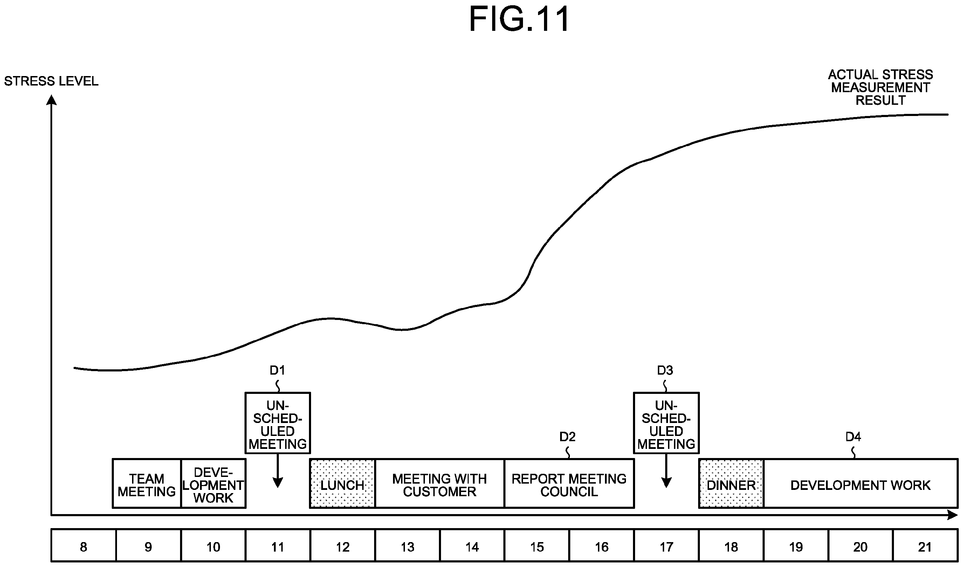

First, a first learning process will be described with reference to FIGS. 10 and 11. The first learning process is a process of learning the relationship between the schedule of the user and the stress of the user.

### (2) Feedback process

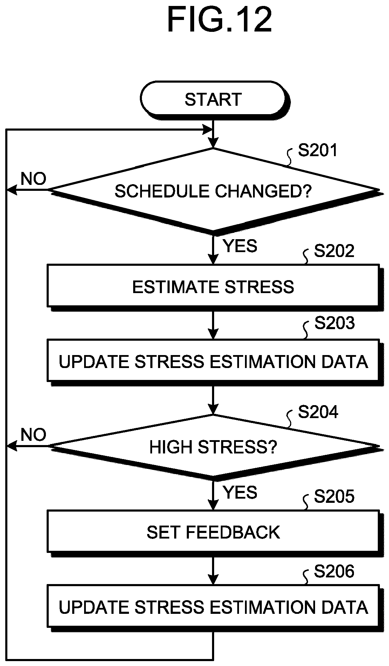

Next, a feedback process will be described with reference to FIGS. 12 to 14. The feedback process is a process of performing feedback to the user based on the stress estimation result.

### (3) Second learning process

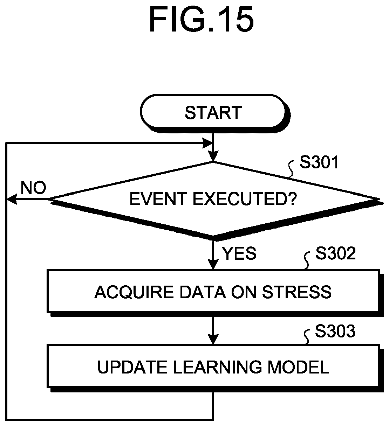

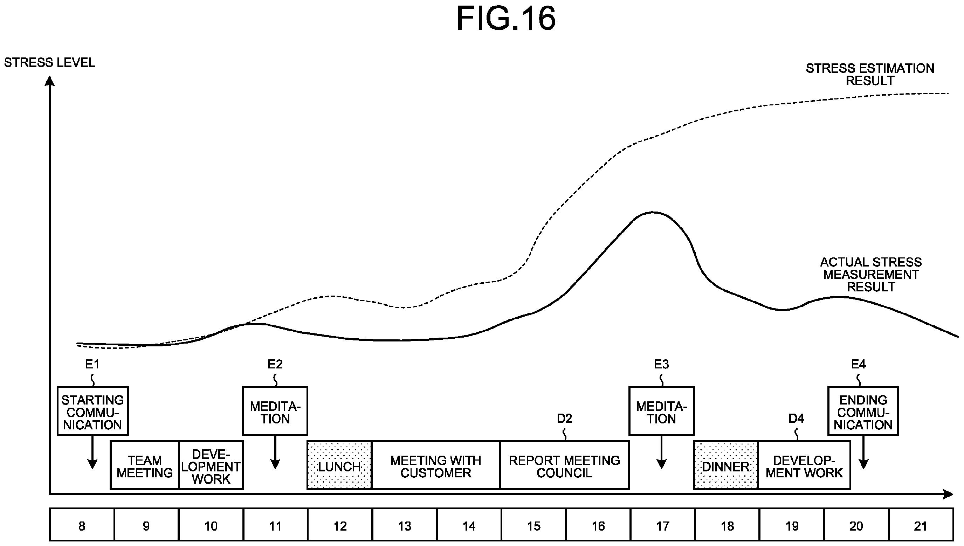

Next, a second learning process will be described with reference to FIGS. 15 and 16. The second learning process is a process of learning the relationship between the feedback to the user and the effect of reducing the stress of the user when the feedback is provided.

### (4) Process when a care target is a group/a plurality of persons

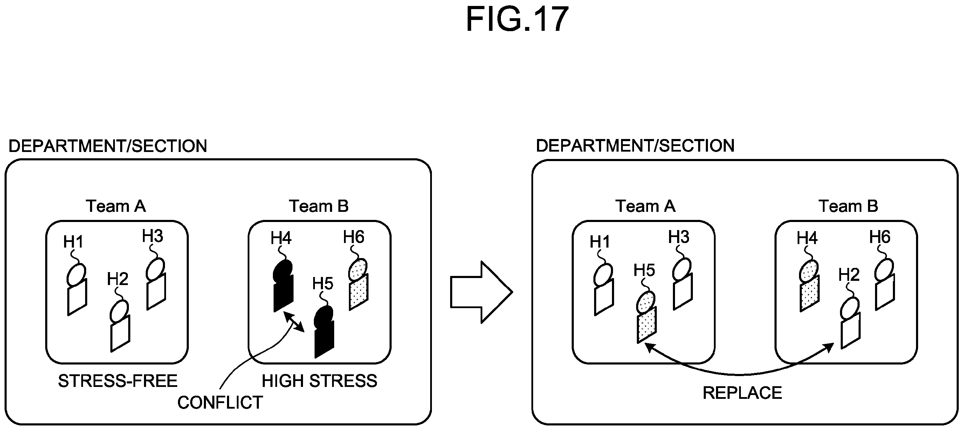

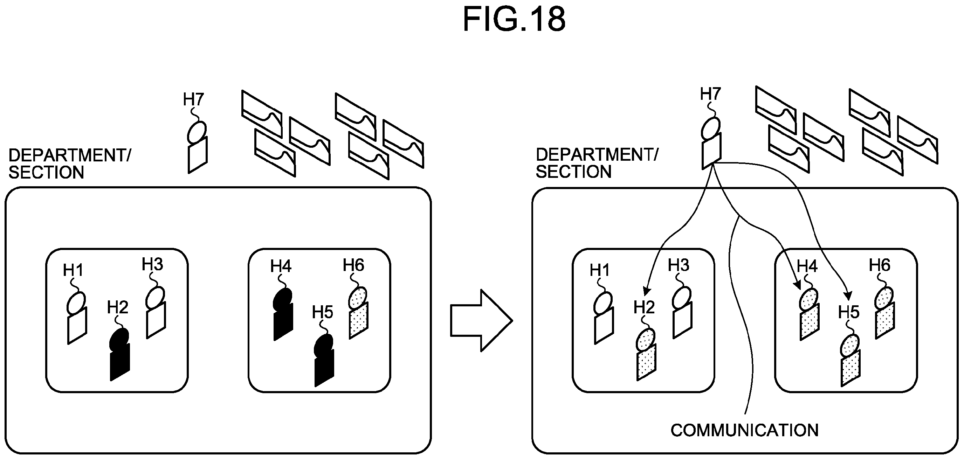

Finally, a process when a stress care target is a group or a plurality of persons will be described with reference to FIGS. 17 and 18.

### <3-1. First learning process>

First, the first learning process will be described.

FIG. 10 is a flowchart illustrating the first learning process according to the present embodiment. As described above, the first learning process is the process of learning the relationship between the schedule of the user and the stress of the user. The first learning process is executed by the management server 10. The first learning process will be described below with reference to a flowchart in FIG. 10.

When the management server 10 is turned on, the control unit 13 of the management server 10 starts the first learning process. The first learning process is executed in parallel with the feedback process and the second learning process.

First, the acquisition unit 131 of the management server 10 determines whether measurement data related to stress of the user has been acquired from the terminal device 20 (Step S101). The measurement data is, for example, the biological information of the user. The biological information may be measured by the sensor unit 26 of the terminal device 20. For example, the biological information may be a user's body temperature, an electrodermal activity, and a pulse. The terminal device 20 transmits the measured biological information to the management server 10 at regular time intervals (e.g., interval of ten seconds). The acquisition unit 131 acquires the measurement data (biological information of the user) from the terminal device 20.

When the measurement data is not acquired from the terminal device 20 (Step S101: No), the acquisition unit 131 repeats Step S101 until the measurement data is acquired.

When the measurement data is acquired from the terminal device 20 (Step S101: Yes), the learning unit 135 of the management server 10 converts the measurement data into the stress value. Various methods can be used for conversion. A conversion method may be a known method or a unique method. The learning unit 135 acquires a converted stress value as the actual measurement data (first actual measurement data) of the stress of the user. FIG. 11 is a diagram illustrating an actual stress measurement result of the user. The actual stress measurement result illustrated in FIG. 11 indicates a transition of the stress value.

The learning unit 135 determines whether there is a predetermined change in the stress value of the user based on the actual stress measurement result (Step S102). For example, the learning unit 135 determines whether or not the stress value of the user has changed beyond a predetermined threshold within a certain period of time in the past. Note that the predetermined change may be simply whether or not the stress value has increased. When there is no change (Step S102: No), the learning unit 135 returns the process to Step S101.

When there is a change (Step S102: Yes), the learning unit 135 associates a change amount of the stress value with the schedule (Step S103). In the example in FIG. 11, it can be seen that the stress value increases at the time of an unscheduled meeting (D1 and D3 in FIG. 11), a report meeting and a council (D2 in FIG. 11), and a development work (D4 in FIG. 11). Therefore, the learning unit 135 associates these events with the change amount of the stress value. Here, the change amount may be a change amount of the stress value within the corresponding event period.

Subsequently, the learning unit 135 scores a stress risk (Step S104). At this time, the learning unit 135 may score the stress risk as illustrated in the following (1) to (3).

### (1) Unscheduled meeting (D1 and D3 in FIG. 11)

As can be seen from the actual stress measurement result in FIG. 11, the stress value slightly increases when the unscheduled meeting is filled in the schedule. It is considered that this is because an unintended event has suddenly occurred. The learning unit 135 scores this by using a predetermined algorithm.

### (2) Report meeting and council (D2 in FIG. 11)

As can be seen from the actual stress measurement result in FIG. 11, the stress value is greatly increased at the report meeting and the council. This is believed to be due to the fact that the report meeting and the council are highly responsible or important. The learning unit 135 scores this by using a predetermined algorithm. For example, the learning unit 135 specifies a level of responsibility or importance from a title and participants of the meeting, and determines a score of the meeting according to the level of responsibility or importance. Note that, in principle, the learning unit 135 may set a schedule in which stress increases every time as a scoring target.

### (3) Development work (D4 in FIG. 11)

As can be seen from the actual stress measurement result in FIG. 11, the stress value continuously increases during the development work. This is believed to be due to the fact that the day's work continues. The learning unit 135 scores this by using a predetermined algorithm.

Subsequently, the learning unit 135 updates the learning model (Step S105). For example, the learning unit 135 learns the relationship between the schedule of the user and the stress of the user based on the schedule of the user and the score of the stress risk associated with the schedule. Then, the learning unit 135 updates the learning model based on the learning result.

Note that the learning model updated by the learning unit 135 may be the first learning model described in <2-3. Learning model>. As described above, the first learning model is the trained model in which at least the schedule information of the user is input and at least the stress information of the user is output. Here, the stress information is, for example, the stress estimation result of the user. The learning unit 135 may train the first learning model so as to output the stress information (e.g., estimation result of stress applied to the user) when the schedule information is input using the data set of the schedule information and the actual stress measurement data of the user in the schedule indicated by the schedule information as learning data.

When the update is completed, the control unit 13 returns the process to Step S101.

### <3-2. Feedback process>

The first learning process has been described above, and now the feedback process will be described below.

FIG. 12 is a flowchart illustrating the feedback process according to the present embodiment. As described above, the feedback process is the process of performing a feedback to the user based on the stress estimation result. The management server 10 executes the feedback process. The feedback process will be described below with reference to the flowchart in FIG. 12.

When the management server 10 is turned on, the control unit 13 of the management server 10 starts the feedback process. The feedback process is executed in parallel with the first learning process and the second learning process.

First, the estimation unit 132 of the management server 10 determines whether the schedule of the user has been changed (Step S201). For example, the estimation unit 132 determines whether the schedule information stored in the storage unit 12 has been changed. When the schedule has not been changed (Step S201: No), the acquisition unit 131 repeats Step S201 until the schedule is changed.

When the schedule has been changed (Step S201: Yes), the estimation unit 132 estimates stress applied to the user based on changed schedule information (Step S202). At this time, the estimation unit 132 estimates the stress applied to the user using the learning model that has learned the relationship between the schedule of the user and the stress applied to the user. The learning model used at this time may be the learning model generated in the first learning process (e.g., first learning model). When the learning model used is the first learning model, the estimation unit 132 acquires the stress information by inputting the schedule information to the first learning model. As described above, the stress information is an estimation result of stress applied to the user.

Then, the estimation unit 132 updates the stress estimation data stored in the storage unit 12 based on the stress estimation result (Step S203).

Next, the determination unit 133 of the management server 10 determines whether the stress applied to the user is high stress (Step S204). For example, the determination unit 133 determines whether a change of the stress of the user indicated in the stress estimation data satisfies a predetermined criterion. For example, it is determined whether an average, maximum value, or minimum value of the change of the stress of the user indicated in the stress estimation data exceeds a predetermined threshold. When the stress applied to the user is not high stress (Step S204: No), the determination unit 133 returns the process to Step S201.

When the stress applied to the user is the high stress (Step S204: Yes), the determination unit 133 determines a feedback to the user based on the stress estimation result. Then, the execution unit 134 of the management server 10 executes the feedback to the schedule of the user based on the determination by the determination unit 133 (Step S205).

### (About feedback)

Here, the feedback may be the schedule setting for changing, adding, or deleting the schedule of the user. In this case, the determination unit 133 may determine the schedule setting based on the stress estimation result.

Note that the schedule setting may be an event setting for reducing the stress of the user. As the event, the following (1) to (5) can be assumed. The event may be a combination of a plurality of events selected from the following (1) to (5). The determination unit 133 sets at least one of the plurality of events including the following (1) to (5) as the feedback to the user based on the stress estimation result.

### (1) Video playback

For example, the determination unit 133 may set, as an event, video playback for the brief meditation. The video for the brief meditation may be, for example, a video focusing on silence (e.g., fragrance of forest, bonfire, ripple, or grandeur). As a result, it is possible to assist the user's meditation.

### (2) Music playback (e.g., music playback for easily implementing a meditation program)

For example, the determination unit 133 may set, as an event, music playback for the user to easily implement the meditation program during the commuting time. The music provided to the user may be healing music. As a result, the user can implement a meditation even during the commuting time.

### (3) Provision of quiet time by noise cancelling technique

For example, the determination unit 133 may set, as an event, provision of quiet time by the noise canceling technique. In this case, the terminal device 20 may include a headphone or earphones capable of executing noise cancellation, and the management server 10 may provide the user with quiet time via the terminal device 20. This allows the user to concentrate on the meditation.

### (4) Aroma presentation by aroma reproduction technique

For example, the determination unit 133 may set, as an event, aroma presentation by aroma reproduction technique. In this case, the terminal device 20 may include an aroma reproduction device capable of reproducing aroma, and the management server 10 may present aroma via the terminal device 20. This allows the user to secure a sense of immersion

### (5) Communication with user by virtual character

For example, the determination unit 133 may set a communication event using a virtual character such as an avatar. In this case, the virtual character may be operated by AI (communication program). The AI may be configured to respond to a question from the user. Since a communication partner is not a person, the user can communicate without feeling much stress.

### (Method for determining feedback)

Various methods can be adopted as a feedback determination method. For example, it is assumed that the feedback to the user is the schedule setting. In this case, the determination unit 133 may determine the schedule setting using the second learning model described above in <2-3. Learning model>. As described above, the second learning model is the trained model that has learned the relationship between the feedback to the user and the effect of reducing the stress of the user when the feedback is provided. More specifically, the second learning model is the trained model that outputs the post-feedback stress estimation result when the feedback information and the pre-feedback stress estimation result are input.

For example, the determination unit 133 selects one of the plurality of schedule setting candidates as the feedback information regarding the input to the second learning model. In addition, the determination unit 133 acquires the stress estimation data updated in Step S203 as the pre-feedback stress estimation result regarding the input to the second learning model. Then, the determination unit 133 acquires the post-feedback stress estimation result by inputting the two pieces of information to the second learning model. The determination unit 133 repeats calculation using the second learning model while changing the schedule setting candidate as an input. The determination unit 133 determines a schedule setting candidate as the schedule setting to feed back to the user, when a desired output (desired post-feedback stress estimation result) is obtained.

### (Working style)

Note that the determination unit 133 may determine the event setting based on the working style. As the working style, (A) Home-based working style and (B) Office-based working style can be assumed.

### (A) Home-based working style

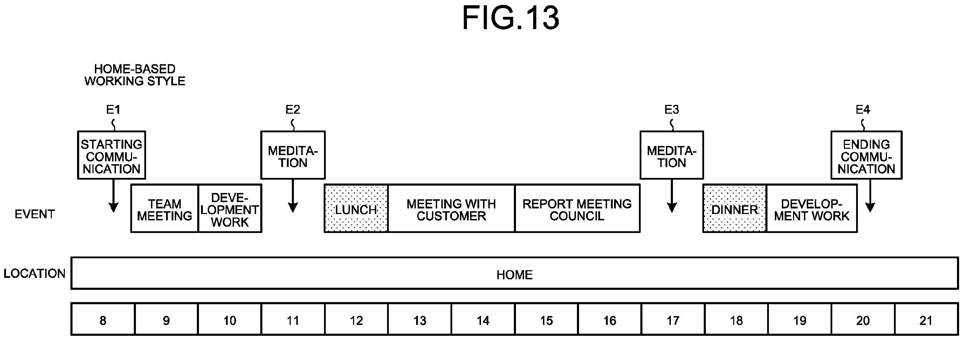

FIG. 13 is a diagram illustrating the event setting in the home-based working style. In the example in FIG. 13, starting communication E1 at the start of work, brief meditations E2 and E3 during work, and ending communication E4 at the end of work are set as events. As the starting communication E1, communication with the user by the virtual character ((5) above) is assumed. Furthermore, the video playback ((1) above), provision of quiet time by the noise canceling technique ((3) above), and/or aroma presentation by the aroma reproduction technique ((4) above) are assumed for the brief meditations E2 and E3.

### (B) Office-based working style

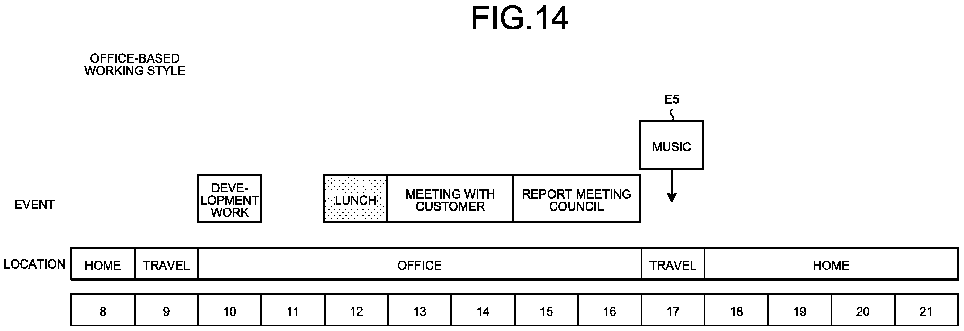

FIG. 14 is a diagram illustrating the event setting in the office-based working style. In the example in FIG. 14, music playback E5 during traveling (commuting) is set as an event. As the music playback E5, music playback ((2) above) can be assumed.

Then, the execution unit 134 incorporates the schedule setting into the schedule information of the user. Note that the feedback determined by the determination unit 133 is not necessarily executed by the execution unit 134. For example, the execution unit 134 of the management server 10 may only transmit the feedback information determined by the determination unit 133 to the terminal device 20 of the user as recommended information for stress reduction.

Next, the estimation unit 132 estimates the stress of the user in the schedule in which the feedback has been provided. Similarly to Step S202, the estimation unit 132 estimates stress applied to the user using the learning model. The learning model used at this time may be the learning model (e.g., first learning model) trained in the first learning process. When the learning model to be used is the first learning model, the estimation unit 132 may acquire the stress information by inputting the schedule information to which the feedback has been provided to the learning model. As described above, the stress information is an estimation result of stress applied to the user. Then, the estimation unit 132 updates the stress estimation data stored in the storage unit 12 based on the stress estimation result (Step S206).

When the update is completed, the control unit 13 returns the process to Step S201.

### <3-3. Second learning process>

The feedback process has been described above, and now the second learning process will be described below.

FIG. 15 is a flowchart illustrating the second learning process according to the present embodiment. As described above, the second learning process is the process of learning the relationship between the feedback to the user and the effect of reducing the stress of the user when the feedback is provided. The second learning process is executed by the management server 10. Hereinafter, the second learning process will be described with reference to the flowchart in FIG. 15.

When the management server 10 is turned on, the control unit 13 of the management server 10 starts the second learning process. The second learning process is executed in parallel with the first learning process and the feedback process.

First, the acquisition unit 131 of the management server 10 determines whether an event related to the feedback has been executed (Step S301). At this time, the acquisition unit 131 may determine whether or not the event has been executed based on whether or not the current time has passed scheduled event time. When the event has not been executed (Step S301: No), the acquisition unit 131 repeats Step S301 until the event is executed.

On the other hand, when the event has been executed (Step S301: Yes), the acquisition unit 131 acquires data regarding the stress of the user (Step S302). The data acquired here is data used for training the learning model. Specifically, the acquisition unit 131 acquires following data (1) to (3). FIG. 16 is a diagram illustrating data acquired by the management server 10 for training the learning model. Hereinafter, the following data (1) to (3) will be described with reference to FIG. 16.

### (1) Feedback information

The acquisition unit 131 acquires the feedback information as stress-related data. The feedback information is information regarding the feedback to the user. The feedback information is, for example, the schedule setting for stress reduction of the user. In the example in FIG. 16, the acquisition unit 131 acquires, as the feedback information, settings of the starting communication E1 at the start of work, the brief meditations E2 and E3 during work, and the ending communication E4 at the end of work.

### (2) Pre-feedback stress estimation result

The acquisition unit 131 acquires the pre-feedback stress estimation result as the stress-related data. The pre-feedback stress estimation result is the estimation result of the stress of the user in the schedule before the feedback (e.g., schedule setting described in (1)) is performed. In the example in FIG. 16, the acquisition unit 131 acquires the stress estimation result indicated by a broken line as the pre-feedback stress estimation result. The stress estimation result indicated by the broken line is the stress estimation result in the schedule before setting the starting communication E1, the brief meditations E2 and E3, and the ending communication E4. Note that the stress estimation result may be the stress estimation data stored in the storage unit 12 in Step S206 of the feedback process described above.

### (3) Second actual measurement data

The acquisition unit 131 acquires the second actual measurement data as the stress-related data. The second actual measurement data is actual measurement data of the stress of the user in the schedule after the feedback (e.g., schedule setting described in (1)) is performed. In the example in FIG. 16, the acquisition unit 131 acquires the actual stress measurement result indicated by a solid line as the second actual measurement data. The actual stress measurement result indicated by the solid line is the actual stress measurement result in the schedule after the setting of the starting communication E1, the brief meditations E2 and E3, and the ending communication E4. Note that the method of acquiring the second actual measurement data may be similar to the method of acquiring the first actual measurement data in the first learning process described above. Specifically, the acquisition unit 131 may acquire the measurement data (e.g., biometric information of the user) from the terminal device 20, and convert the acquired measurement data into the stress value according to a predetermined criterion to acquire the second actual measurement data.

Next, the learning unit 135 of the management server 10 updates the learning model (Step S303). For example, the learning unit 135 learns the relationship between the feedback to the user and the effect of reducing the stress of the user when the feedback is provided based on the data acquired in Step S302. Then, the learning unit 135 updates the learning model based on the learning result.

Note that the learning model updated by the learning unit 135 may be the second learning model described in <2-3. Learning model>. As described above, the second learning model is the trained model having at least the feedback information and the pre-feedback stress estimation result as the input and at least the post-feedback stress estimation result as the output.

The learning unit 135 may train the second learning model so as to output the post-feedback stress estimation result when the feedback information and the pre-feedback stress estimation result are input using the data set of the feedback information, the pre-feedback stress estimation result, and the second actual measurement data as learning data. The post-feedback stress estimation result is an estimation result of the stress of the user in the schedule after the feedback is provided.

When the update is completed, the control unit 13 returns the process to Step S301.

### <3-4. Process when a care target is a group/a plurality of persons>

The second learning process has been described above, and now the process when the stress care target is a group/a plurality of persons will be described below.

With the spread of home-based working, it is difficult to grasp relationships and stress factors among members of a team. Here, by expanding the target of the stress estimation described above to a group or a plurality of people, a stress manager such as a manager of a company can appropriately respond even when many members are working at home.

FIG. 17 is a diagram illustrating an example of the process when the care target is a group/a plurality of persons. The left side of FIG. 17 illustrates a situation of a department/section of a company before improvement, and the right side illustrates a situation of the department/section after improvement. There are two teams, Team A and Team B, in the department/section. Three employees belong to each of Team A and Team B. Employees H1, H2, and H3 belong to Team A, and employees H4, H5, and H6 belong to Team B. Team A has good personal relationship, and the team is in a stress-free state. However, in Team B, the employees H4 and H5 are always in conflict with each other and are in a highly stressed state. In FIG. 17, darker colored employees are more stressed.

The management server 10 repeatedly performs stress estimation of the plurality of employees (employees H1 to H6) at a predetermined timing. Then, the management server 10 transmits the stress estimation result of each of the plurality of employees to the stress manager. The stress manager checks the stress estimation result and replaces/assigns a person. In the example in FIG. 17, the manager replaces one (employee H5) of the conflicting employees in Team B with one employee (employee H2) in Team A. As a result, since the stress manager can appropriately allocate personnel, stress of the employees can be reduced. In the example in FIG. 17, it can be seen that the color of the employee is lighter, and the stress of the employee is reduced. Note that the management server 10 may be configured to present information on countermeasures (e.g., person replacement/assignment) to the stress manager based on the stress estimation result.

FIG. 18 is a diagram illustrating another example of the process when the care target is a group/a plurality of persons. The left side of FIG. 18 illustrates a situation of a department/section of a company before improvement, and the right side illustrates a situation of the department/section after improvement. As in the case in FIG. 17, there are two teams, Team A and Team B, in the department/section. Three employees belong to each of Team A and Team B. Employees H1, H2, and H3 belong to Team A, and employees H4, H5, and H6 belong to Team B. In the example in FIG. 18, highly stressed persons are scattered in both Team A and Team B. In the example in FIG. 18, the employees H2, H4, and H5 are highly stressed. In FIG. 17, darker colored employees are more stressed.

The management server 10 repeatedly performs stress estimation of the plurality of employees (employees H1 to H6) at a predetermined timing. Then, the management server 10 transmits the stress estimation result of each of the plurality of employees to the stress manager (e.g., boss H7). The stress manager looks at the stress estimation result and performs communication (e.g., problem consultation) and improvement (e.g., adjustment of vacations and tasks). As a result, the stress manager can appropriately communicate with a highly-stressed employee, so that the stress of the employee can be reduced. In the example in FIG. 18, it can be seen that the color of the employee is lighter, and the stress of the employee is reduced. Note that the management server 10 may be configured to present information on countermeasures to the stress manager based on the stress estimation result. As the information on the countermeasures, for example, information indicating with whom to communicate and information on schedule adjustment of a highly-stressed person is assumed.

### <<4. Modifications>>

The above-described embodiments are examples, and various modifications and applications are possible.

### <4-1. Modification regarding stress estimation>

For example, in the above-described embodiment, the management server 10 estimates the stress applied to the user based on the schedule information, but the management server 10 may estimate the stress applied to the user using not only the schedule information but also real-time information. At this time, the management server 10 may use the learning model for stress estimation. In this case, the learning model may be configured to receive the real-time information in addition to the schedule information.

Here, the real-time information is information obtained in real time. As the real-time information, the following (1) to (5) are assumed. The management server 10 estimates stress applied to the user based on the schedule information and at least one of a plurality of pieces of real-time information including the following (1) to (5).

### (1) Location information

The real-time information may include location information indicating a place where the user is currently located. The location information is, for example, information indicating a place where the user is located, such as "office" or "home". For example, the management server 10 acquires position information such as GPS information from the terminal device 20 of the user, and identifies a place where the user is currently located based on the acquired position information. It is considered that the stress applied to the user varies depending on the location. For example, it is assumed that the stress level is higher when the user is in the office than when the user is at home. Therefore, by adding the location information to the schedule information, the stress estimation accuracy can be improved.

### (2) Environment information

The real-time information may include the environment information of a place where the user is located. The environment information is, for example, weather information, temperature information, and humidity information. For example, the management server 10 acquires information on the current position of the user from the terminal device 20 of the user, and acquires the weather information of a place where the user is located from a server that transmits the weather information via a network. In addition, the management server 10 acquires information on the temperature and humidity measured by the terminal device 20 from the terminal device 20 of the user. It is considered that the stress applied to the user changes depending on weather, temperature, and humidity. Therefore, by adding the weather information, the temperature information, and/or the humidity information to the schedule information, the stress estimation accuracy can be improved.

The environment information may include carbon dioxide concentration information. For example, the management server 10 acquires information on temperature and humidity measured by the terminal device 20 from the terminal device 20 of the user. It is considered that the stress applied to the user also changes depending on the concentration of carbon dioxide. Therefore, by adding the carbon dioxide concentration information to the schedule information, the stress estimation accuracy can be improved. When the estimation result of the stress applied to the user satisfies a predetermined criterion and the carbon dioxide is higher than a predetermined value, the management server 10 may set an event of ventilation or outing as feedback to the user. The stress of the user caused by the concentration of carbon dioxide can be reduced.

### (3) Season information

The real-time information may include season information of a place where the user is located. For example, the management server 10 acquires information of the current position of the user from the terminal device 20 of the user, and identifies a season of the place where the user is located. It is considered that the stress applied to the user changes depending on the season. Therefore, by adding the season information to the schedule information, the stress estimation accuracy can be improved.

### (4) Vital information

The real-time information may include vital information (biological information) of the user. The vital information is, for example, information on the user's respiration, pulse, blood pressure, body temperature, or consciousness level. For example, the management server 10 acquires information on respiration, pulse, blood pressure, body temperature, and/or a consciousness level measured by the terminal device 20 from the terminal device 20 of the user. By adding the vital information to the schedule information, the stress estimation accuracy can be improved.

### (5) Behavior information

The real-time information may include behavior information of the user. The behavior information is, for example, determination information of negative emotion based on keyboard input or e-mail input by the user. Furthermore, the behavior information may be change information of the user's voice tone. Furthermore, factory oscillation may be detection information of the user's sigh, detection information of the user's tongue clicking, or detection information of the user talking to oneself. By adding the behavior information to the schedule information, the stress estimation accuracy can be improved.

### <4-2. Modification regarding determination of feedback>

For example, in the above-described embodiment, the management server 10 makes the determination regarding the feedback based on the stress estimation result, but the management server 10 may make the determination regarding the feedback using not only the stress estimation result but also situation information of the user. Here, the situation information is information indicating a situation in which the user is placed. The situation information is, for example, the location information indicating a place where the user is located. For example, the location information is information such as "Office" or "Home". For example, it is considered that it is easy for the user to implement the meditation event when the user is at home, but it is difficult to implement the meditation event when the user is in the office. Therefore, the management server 10 changes the feedback to the user according to the place where the user is located.

Furthermore, the situation information may be activity information indicating an activity performed by the user. For example, the activity information is information on "commuting", "desk work", and "service". For example, it is considered that it is easy for the user to implement the meditation event during desk work, but it is difficult for the user to implement the meditation event during commuting. Therefore, the management server 10 changes the feedback to the user according to the activity performed by the user.

### <4-3. Modification regarding stress factor identification>

In the above-described embodiment, the management server 10 estimates the stress applied to the user based on the schedule information, but the management server 10 may perform not only the estimation of the stress but also identification of a stress factor based on the schedule information. For example, the management server 10 may identify the stress factor based on information such as a conference title, participants, and conference content (agenda) included in the schedule information.

Note that the management server 10 may be configured to set a counseling event as the feedback to the user when the stress factor cannot be identified. The counseling may be performed by the virtual character using AI. The stress factor identification can lead to stress reduction of the user.

### <4-4. Other modifications>

In the above-described embodiment, the management server 10 is the information processing apparatus (computer) on a network, but the management server 10 may not necessarily be the information processing apparatus on a network. For example, the management server 10 may be a local information processing apparatus (computer). In addition, some or all of the functions (e.g., the acquisition unit 131 to the transmission unit 136) of the management server 10 of the present embodiment may be included in an information processing apparatus other than the management server 10. For example, the terminal device 20 may have some or all of the functions of the management server 10 of the present embodiment. Then, the terminal device 20 may execute the above-described process (first learning process, feedback process, second learning process, or process when the care target is a group/a plurality of persons).

The control device that controls the management server 10 or the terminal device 20 of the present embodiment may be realized by a dedicated computer system or may be realized by a general-purpose computer system.

For example, a communication program for executing the above-described operation is stored and distributed in a computer-readable recording medium such as an optical disk, a semiconductor memory, a magnetic tape, or a flexible disk. Then, for example, the program is installed in a computer, and the above-described processes are executed to configure the control device. Here, the control device may be a device (e.g., personal computer) outside the management server 10 and the terminal device 20. Furthermore, the control device may be a device inside the management server 10 or the terminal device 20 (e.g., the control unit 13 or the control unit 23).

In addition, the above communication program may be stored in a disk device included in a server device on a network such as the Internet so that the communication program can be downloaded to the computer. In addition, the above-described functions may be realized by cooperation of an operating system (OS) and application software. In this case, a portion other than the OS may be stored in a medium and distributed, or a portion other than the OS may be stored in a server device and downloaded to the computer.

Among the processes described in the above embodiments, all or part of the processes described as being performed automatically can be performed manually, or all or part of the processes described as being performed manually can be performed automatically by a known method. In addition, the processing procedure, specific name, and information including various data and parameters illustrated in the above document and the drawings can be arbitrarily changed unless otherwise specified. For example, various types of information illustrated in each drawing are not limited to the illustrated information.

In addition, each component of each device illustrated in the drawings is functionally conceptual, and is not necessarily physically configured as illustrated in the drawings. In other words, a specific form of distribution and integration of each device is not limited to the illustrated form, and all or a part thereof can be functionally or physically distributed and integrated in an arbitrary unit according to various loads, usage conditions, and the like.

In addition, the above-described embodiments can be appropriately combined in a region in which the processing content do not contradict each other. Furthermore, the order of each step illustrated in the flowchart of the above-described embodiments can be appropriately changed.

Furthermore, for example, the present embodiment can be implemented as any configuration constituting an apparatus or a system, for example, a processor as a system large scale integration (LSI) or the like, a module using a plurality of processors or the like, a unit using a plurality of modules or the like, a set obtained by further adding other functions to a unit, or the like (i.e., configuration of a part of device).

Note that, in the present embodiment, the system means a set of a plurality of components (devices, modules (parts), etc.), and it does not matter whether or not all the components are in the same housing. Therefore, a plurality of devices housed in separate housings and connected via a network and one device in which a plurality of modules is housed in one housing are both systems.

Furthermore, for example, the present embodiments can adopt a configuration of cloud computing in which one function is shared and processed by a plurality of devices in cooperation via a network.

### «5. Conclusion»

As described above, according to one embodiment of the present disclosure, the management server 10 estimates stress applied to the user based on the schedule information of the user, and makes the determination regarding feedback related to the schedule of the user based on the estimation result. For example, the management server 10 can insert an event such as the brief meditation or communication into the schedule of the user. The user implements the event. As a result, the management server 10 can appropriately reduce the stress of the user according to the estimation result and also estimate the stress applied to the user. In addition, it is also possible to more effectively reduce the stress by identifying the plan (cause) in which the stress greatly changes based on the change of the stress estimation result and inserting an event before and after the plan. Furthermore, by executing the communication program also at the start/end time of the work, it is possible to make the user aware of the start/end time of the work. Still more, it is also possible to suppress long work by the user and encourage the way of working in which work and private are clearly divided.

Although the embodiments of the present disclosure have been described above, the technical scope of the present disclosure is not limited to the above-described embodiments as it is, and various modifications can be made without departing from the gist of the present disclosure. In addition, the components of different embodiments and modifications may be appropriately combined.

Note that the effects of each embodiment described in the present specification are merely examples and not limited thereto, and other effects may be provided.

The present technology can also have the following configurations.
(1) An information processing apparatus comprising:
   an estimation unit configured to estimate stress applied to a user based on schedule information of the user; and
   a determination unit configured to make a determination of a feedback related to a schedule of the user based on an estimation result of the stress.
(2) The information processing apparatus according to (1), wherein
   the feedback is a schedule setting for changing, adding, or deleting the schedule of the user, and
   the determination unit determines the schedule setting based on the estimation result of the stress.
(3) The information processing apparatus according to (2), wherein
   the schedule setting is a setting of an event for reducing the stress of the user, and
   the determination unit determines the setting of the event based on the estimation result of the stress.
(4) The information processing apparatus according to (3), wherein
   the event includes playback of a predetermined video.
(5) The information processing apparatus according to (3) or (4), wherein
   the event includes at least one of provision of quiet time by a noise canceling technique and aroma presentation by an aroma reproduction technique.
(6) The information processing apparatus according to any one of (3) to (5), wherein
   the event includes communication with the user by a virtual character.
(7) The information processing apparatus according to any one of (3) to (6), wherein
   the determination unit sets an event of ventilation or outing when the estimation result of the stress satisfies a predetermined criterion and a carbon dioxide concentration in a place where the user is located is not less than a predetermined value.
(8) The information processing apparatus according to any one of (2) to (7), comprising
   an execution unit configured to incorporate the schedule setting into the schedule information of the user.
(9) The information processing apparatus according to any one of (1) to (8), wherein
   the estimation unit estimates the stress applied to the user based on the schedule information and real-time information obtained in real time.
(10) The information processing apparatus according to (9), wherein
   the real-time information includes at least one of weather information, temperature information, humidity information, and carbon dioxide concentration information in a place where the user is located.
(11) The information processing apparatus according to (9) or (10), wherein
   the real-time information includes location information indicating a place where the user is currently located.
(12) The information processing apparatus according to any one of (9) to (11), wherein
   the real-time information includes at least one of vital information and behavior information of the user.
(13) The information processing apparatus according to (12), wherein
   the real-time information includes the behavior information of the user, and
   the behavior information of the user includes information on determination of negative emotion based on a keyboard input or an e-mail input by the user.
(14) The information processing apparatus according to (12) or (13), wherein
   the real-time information includes the behavior information of the user, and
   the behavior information of the user includes at least one of voice tone change information, sighing detection information, tongue-clicking detection information, and self-talk detection information of the user.
(15) The information processing apparatus according to any one of (1) to (14), wherein
   the estimation unit estimates the stress applied to the user by using a first learning model that has learned a relationship between the schedule of the user and the stress applied to the user.
(16) The information processing apparatus according to (15), further comprising:
   an execution unit configured to execute the feedback to the schedule of the user based on the determination by the determination unit;
   an acquisition unit configured to acquire actual measurement data of the stress of the user in a schedule after the feedback is provided; and
   a learning unit configured to train a second learning model that learns a relationship between the feedback and an effect of reducing the stress, wherein
   the learning unit trains the second learning model based on stress estimation data of the user in the schedule before the feedback is provided and the actual measurement data of the stress of the user in the schedule after the feedback is provided, and
   the determination unit uses the second learning model to determine the feedback.
(17) The information processing apparatus according to any one of (1) to (16), wherein
   the schedule information includes situation information indicating a situation in which the user is placed, and
   the determination unit determines the feedback based on the estimation result of the stress and the situation information.
(18) The information processing apparatus according to (17), wherein
   the situation information includes at least one of information indicating a place where the user is located and information indicating an activity performed by the user.
(19) An information processing method causing one or a plurality of processors to implement:
   estimating stress applied to a user based on schedule information of the user, and
   determining a feedback related to a schedule of the user based on an estimation result of the stress.
(20) A program causing
   a computer to function as:
   an estimation unit configured to estimate stress applied to a user based on schedule information of the user; and
   a determination unit configured to determine a feedback related to a schedule of the user based on an estimation result of the stress.

### Reference Signs List

- 1: MANAGEMENT SYSTEM

- 10: MANAGEMENT SERVER
- 20: TERMINAL DEVICE
- 11, 21: COMMUNICATION UNIT
- 12, 22: STORAGE UNIT
- 13, 23: CONTROL UNIT
- 24: INPUT UNIT
- 25: OUTPUT UNIT
- 26: SENSOR UNIT
- 131: ACQUISITION UNIT
- 132: ESTIMATION UNIT
- 133: DETERMINATION UNIT
- 134: EXECUTION UNIT
- 135: LEARNING UNIT
- 136: TRANSMISSION UNIT

## Claims

1. An information processing apparatus comprising:
an estimation unit configured to estimate stress applied to a user based on schedule information of the user; and
a determination unit configured to make a determination of a feedback related to a schedule of the user based on an estimation result of the stress.

2. The information processing apparatus according to claim 1, wherein
the feedback is a schedule setting for changing, adding, or deleting the schedule of the user, and
the determination unit determines the schedule setting based on the estimation result of the stress.

3. The information processing apparatus according to claim 2, wherein
the schedule setting is a setting of an event for reducing the stress of the user, and
the determination unit determines the setting of the event based on the estimation result of the stress.

4. The information processing apparatus according to claim 3, wherein
the event includes playback of a predetermined video.

5. The information processing apparatus according to claim 3, wherein
the event includes at least one of provision of quiet time by a noise canceling technique and aroma presentation by an aroma reproduction technique.

6. The information processing apparatus according to claim 3, wherein
the event includes communication with the user by a virtual character.

7. The information processing apparatus according to claim 3, wherein
the determination unit sets an event of ventilation or outing when the estimation result of the stress satisfies a predetermined criterion and a carbon dioxide concentration in a place where the user is located is not less than a predetermined value.

8. The information processing apparatus according to claim 2, comprising
an execution unit configured to incorporate the schedule setting into the schedule information of the user.

9. The information processing apparatus according to claim 1, wherein
the estimation unit estimates the stress applied to the user based on the schedule information and real-time information obtained in real time.

10. The information processing apparatus according to claim 9, wherein
the real-time information includes at least one of weather information, temperature information, humidity information, and carbon dioxide concentration information in a place where the user is located.

11. The information processing apparatus according to claim 9, wherein
the real-time information includes location information indicating a place where the user is currently located.

12. The information processing apparatus according to claim 9, wherein
the real-time information includes at least one of vital information and behavior information of the user.

13. The information processing apparatus according to claim 12, wherein
the real-time information includes the behavior information of the user, and
the behavior information of the user includes information on determination of negative emotion based on a keyboard input or an e-mail input by the user.

14. The information processing apparatus according to claim 12, wherein
the real-time information includes the behavior information of the user, and
the behavior information of the user includes at least one of voice tone change information, sighing detection information, tongue-clicking detection information, and self-talk detection information of the user.

15. The information processing apparatus according to claim 1, wherein
the estimation unit estimates the stress applied to the user by using a first learning model that has learned a relationship between the schedule of the user and the stress applied to the user.

16. The information processing apparatus according to claim 15, further comprising:
an execution unit configured to execute the feedback to the schedule of the user based on the determination by the determination unit;
an acquisition unit configured to acquire actual measurement data of the stress of the user in a schedule after the feedback is provided; and
a learning unit configured to train a second learning model that learns a relationship between the feedback and an effect of reducing the stress, wherein
the learning unit trains the second learning model based on stress estimation data of the user in the schedule before the feedback is provided and the actual measurement data of the stress of the user in the schedule after the feedback is provided, and
the determination unit uses the second learning model to determine the feedback.

17. The information processing apparatus according to claim 1, wherein
the schedule information includes situation information indicating a situation in which the user is placed, and
the determination unit determines the feedback based on the estimation result of the stress and the situation information.

18. The information processing apparatus according to claim 17, wherein
the situation information includes at least one of information indicating a place where the user is located and information indicating an activity performed by the user.

19. An information processing method causing one or a plurality of processors to implement:
estimating stress applied to a user based on schedule information of the user, and
determining a feedback related to a schedule of the user based on an estimation result of the stress.

20. A program causing
a computer to function as:
an estimation unit configured to estimate stress applied to a user based on schedule information of the user; and
a determination unit configured to determine a feedback related to a schedule of the user based on an estimation result of the stress.
